# EUROPEAN PATENT SPECIFICATION

(11) **EP 3 603 631 B1**
(45) Date of publication and mention of the grant of the patent: **29.11.2023**
(21) Application number: 19199336.9
(22) Date of filing: 14.12.2012
(51) Int. Cl.: A61K 31/198, A61P 1/00, A61K 31/717, A61K 31/721, A61K 36/68

(54) **METHODS AND COMPOSITIONS FOR THE TREATMENT OF DIVERTICULOSIS**
VERFAHREN UND ZUSAMMENSETZUNGEN ZUR BEHANDLUNG VON DIVERTIKULOSE
PROCÉDÉ ET COMPOSITIONS POUR LE TRAITEMENT D'UNE DIVERTICULOSE

(30) Priority: 19.12.2011 US 201161630831 P
(43) Date of publication of application: 05.02.2020
(62) Divisional of application: 12860587.0
(73) Proprietor: Emmaus Medical, Inc., Torrance, CA 90503 (US)
(72) Inventor: NIIHARA, Yutaka, Rolling Hills Estates, CA California 90274 (US)
(74) Representative: Boult Wade Tennant LLP

(56) References cited:
- US-A1- 2004 005 304
- US-A1- 2004 096 479
- US-A1- 2005 058 671
- EVANS M A ET AL: "INTESTINAL FUELS: GLUTAMINE, SHORT-CHAIN FATTY ACIDS, AND DIETARY FIBER", JOURNAL OF THE AMERICAN DIETETIC ASSOCIATION, THE ASSOCIATION, CHICAGO, IL, US, vol. 92, no. 10, 1 October 1992 (1992-10-01), page 1239, XP009045257, ISSN: 0002-8223

## Description

### Field of the Invention

The present invention is directed to compositions for use in methods for the treatment of diverticulosis. It is more specifically directed to compositions including L-glutamine, or its salts, and uses of such compositions in the treatment of diverticulosis.

### Background of the Invention

Diverticulosis refers to a condition where pouches *(i.e.,* diverticula) form along the colon wall. The pouches are typically 5 to 10 millimeters in diameter and are formed in approximately 50 percent of individuals 60 years old or older.

The etiology of diverticulosis is not well understood. Current theory focuses on observed nutritional differences between cultures: Those cultures that eat a high fiber diet (*e*.*g*., Asia and Africa) have a very low incidence of diverticulosis; those that eat a low fiber diet (*e.g.,* United States and Europe) have a high incidence of diverticulosis. Certain physicians believe that the colon of a person eating a diet low in fiber must exert more pressure than usual to move waste through his colon. The relative high pressure distorts weak spots in the colon where blood vessels pass through the muscle layer of the bowel wall, thereby forming pouches.

Many cases of diverticulosis go undiscovered, because the condition alone does not cause symptoms. Diverticulosis is typically diagnosed after a patient experiences complications, such painful diverticular disease or diverticulitis. In other cases, it is discovered during a screening examination (e.g., colonoscopy).

Diverticulosis is usually treated using a combination of nutritional guidelines and medication. A patient may follow a diet regimen involving ingestion of high fiber foods with the objective of consuming 30 to 35 grams of fiber per day. Antispasmodic drugs may be prescribed to relax muscles around the digestive tract. Painkillers may be administered where diverticulitis or other complications of diverticulosis have occurred.

There is a need in the art for additional compositions and methods that can be used to treat diverticulosis. That is an object of the present invention.

US2004005304 describes therapeutic compositions and methods for treating neurological disorders and associated gastrointestinal conditions using enhancer molecules. These enhancer molecules comprise therapeutically effective amounts of metals, amino acids, polypeptides, saccharides, probiotics, and combinations thereof to enhance expression of genes, and/or enzymatic activity of gastrointestinal proteins.

US2005058671 describes a dietary supplement and methods for the manufacture and administration of the same for the treatment and/or prevention of digestive disorders and digestive tract-related disorders.

US2004096479 describes an ultra-high fiber supplement that promotes satiety, caloric reduction, and weight loss. The supplement comprises guar, oat, psyllium, locust bean gum, pectin, green tea, multi-anthocyanadins, pyridoxine, and folic acid.

Evans and Shronts, J. AM. Diet. Assoc., (1992), 92; 1239-1246, 1249 describes glutamine, short-chain fatty acids and dietary fiber as intestinal fuels.

### Brief Description of the Figures

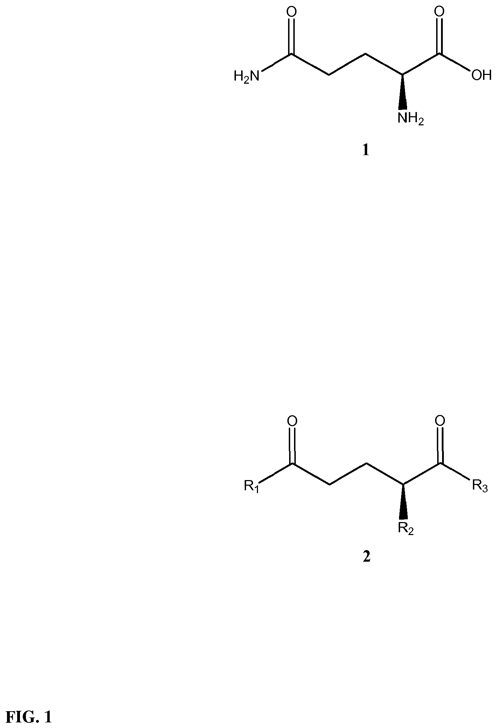

FIG. 1 shows L-glutamine (1) and L-glutamine salts and derivatives (2).

### Summary of the Invention

The present invention is directed to compositions for use in methods for the treatment of diverticulosis. It is more specifically directed to compositions comprising L-glutamine or an L-glutamine salt for use in methods of treating diverticulosis.

In a further aspect, the present invention also provides a composition for use in a method of treating diverticulosis, wherein the composition includes L-glutamine or an L-glutamine salt and a fiber supplement.

### Detailed Description of the Invention

The present invention is directed to compositions for use in methods of treating diverticulosis. It is more specifically directed to compositions including L-glutamine or an L-glutamine salt for use in methods of treating diverticulosis.

The structure of L-glutamine is shown in **FIG. 1**, compound **1**. L-glutamine salts and derivatives are shown as compound **2** in **FIG. 1**. Nonlimiting L-glutamine salts and derivatives have the following substituents in reference to compound **2**:
R₁ is NH₂;
R₂ is NH₂, NH₃⁺, NH₃Br, NH₃OPO₃H, NH₃OC(O)CH₃ (*i.e.*, acetate salt), NH₃OC(O)CHCHCO₂H *(i.e.,* fumarate salt- *trans* olefin), NH₃OC(O)CH(OH)CH(OH)CO₂H *(i.e.,* tartrate salt), NH₃OC(O)CHCHCO₂H (*i*.*e*., maleate salt - *cis* olefin), NH₃OC(O)CH₂-C(OH)(CO₂H)CH₂CO₂H (*i*.*e*., citrate salt), NH₃OC(O)CO₂H (*i*.*e*., oxalate salt), NH₃OS(O)₂OH *(i.e.,* methanesulfonate salt), NH₃OS(O)₂CeH₄CH₃ (*i.e.*, *p*-toluenesulfonate salt), and, NH₃OC(O)C(O)CH₂CH₂CO₂H (*i.e.*, alpha-ketoglutarate salt).
R₃ is OH, O⁻, ONa, OK, OCa, OLi, ONH₂(CH₂C₆H₅)CH₂CH₂NHCH₂C₆H₆ (*i.e.*, benzathine salt), ON(CH₂CH₃)₂CH₂CH₂OC(O)C₆H₃ClNH₂ (*i*.*e*., chloroprocaine salt), ON(CH₃)₃CH₂CH₂OH (*i*.*e*., choline salt), ONH₂(CH₂CH₂OH)₂ (*i*.*e*., diethanolamine salt), ONH₃CH₂CH₂OH (*i.e.*, ethanolamine salt), ONH₃CH₂CH₂NH₂ (*i.e.*, ethyldiamine salt), ONH₂(CH₃)CH₂CH(OH)CH(OH)CH(OH)CH(OH)CH₂OH (*i*.*e*., meglumine salt), ONH₃C(CH₂OH)₃ (*i.e.*, tromethamine salt), ONH₃C(CH₃)₃ (*i.e.*, tertiary-butylamine salt), ON(CH₂CH₃)₂CH₂CH₂OC(O)C₆H₄NH₂ (*i.e.*, procaine salt), NHCH(CH₃)CO₂H, NHCH(CH(CH₃)₂)CO₂H, NHCH(CH(CH₃)(CH₂CH₃))CO₂H, NHCH(CH₂CH(CH₃)₂)CO₂H, NHCH(CH₂CH₂SCH₃)CO₂H, NHCH(CH₂C₆H₅)CO₂H, NHCH(CH₂C₆H₅OH)CO₂H, NHCH(CH₂C₈H₆N)CO₂H, NHCH₂CO₂H, NHCH(CH₂CH₂C(O)NH₂)CO₂H, NHCH(CH₂C(O)NH₂)CO₂H, NHCH(CH(OH)CH₃)CO₂H, NHCH(CH₂OH)CO₂H, NHCH(CH₂CH₂CO₂H)CO₂H, NHCH(CH₂CO₂H)CO₂H.

Typically, a single compound (e.g., L-glutamine) is administered to a person seeking treatment for diverticulosis. In certain cases, however, a mixture of two or more compounds may be administered. For instance, L-glutamine may be administered with one or more L-glutamine salts.

L-glutamine, its salts, or mixtures for use according to the invention may be administered in any suitable way. For example, in certain cases it is administered as an aqueous solution. The solution may only have L-glutamine or its salts as dissolved ingredients, or it may include other pharmaceutically acceptable compounds. Nonlimiting examples of such compounds include buffers and flavorants.

Other, non-limiting examples of ways in which L-glutamine, its salts, or mixtures may be administered include: as a suspension in a liquid (*e*.*g*., water or juice); as a solid, either as a powder or in another form (*e.g.*, pill or capsule); as a mixture with food (*e.g.*, yogurt). As with the aqueous solution, the preceding administration forms may include other pharmaceutically acceptable ingredients.

The amount of L-glutamine or its salts administered for use in accordance with the present invention range from 0.05 g/kg body weight to 10.0 g/kg body weight. Oftentimes, the amount ranges from 0.10 g/kg body weight to 8.0 g/kg body weight. In certain cases, the amount ranges from 0.15 g/kg body weight to 7.0 g/kg body weight.

L-glutamine or its salts are typically administered to a person once per day. The compounds may, however, be administered more than once per day where indicated.

Compositions for use in accordance with the present invention may be combined with other methods used to treat diverticulosis. For instance, the compositions may be administered as part of a dietary regimen focused on the inclusion of increased fiber.

Compositions for use in accordance with the present invention may also be administered and/or combined with a fiber supplement such as CITRUCEL, BENEFIBER and METAMUCIL. The fiber supplement may include soluble and/or insoluble fiber, or the supplement may be entirely composed of soluble and/or insoluble fiber. Nonlimiting examples of fiber with which the compositions can be combined include: methylcellulose; wheat dextrin natural soluble fiber; and, psyllium fiber. The fiber may also include combinations of the foregoing types of fibers.

Combinations of the compositions and fiber may be of any suitable form. The combination may be, for example, in the form of a powder, a capsule, a caplet, a chewable tablet. It may also be included in other supplement forms, such as drinks or gels.

Compositions for use according to the present invention may be used to treat diverticulosis as determined by a number of different methods to image colon pouches. Nonlimiting examples of imaging methods include: colonoscopy; computed tomography; and, x-ray based on a barium enema.

After administration of L-glutamine or its salts for three months, four months, five months, six months, seven months, eight months, nine months, ten months, eleven months or twelve months, the number of pouches observed in a patient's colon have been reduced by at least twenty-five (25) percent. In certain cases, the number of pouches has been reduced by at least fifty (50) percent or seventy-five (75) percent. In other cases, the number of pouches has been reduced by at least eighty (80) percent, eighty-five (85) percent, ninety (90) percent, or ninety-five (95) percent.

### Experimental Results

A person had a number of diverticula as observed by colonoscopy. The person ingested 30 g of L-glutamine per day as an aqueous solution. After twelve months of L-glutamine administration, the number of diverticula had been reduced by ninety (90) percent.

## Claims

1. L-glutamine or an L-glutamine salt for use in a method of treating diverticulosis, wherein the method comprises ingestion of 0.05 g/kg body weight to 10.0 g/kg body weight of the L-glutamine or L-glutamine salt per day by a person who has diverticulosis, wherein the L-glutamine or L-glutamine salt is administered once per day.

2. L-glutamine or an L-glutamine salt for use in a method of treating diverticulosis, wherein the method comprises ingestion of 0.05 g/kg body weight to 10.0 g/kg body weight of the L-glutamine or L-glutamine salt per day by a person who has diverticulosis, wherein the L-glutamine or L-glutamine salt is administered more than once per day.

3. L-glutamine for use in a method according to claim 1 or claim 2.

4. L-glutamine for use in a method according to claim 3, wherein the L-glutamine is ingested as part of an aqueous solution or suspension.

5. L-glutamine for use in a method according to claim 4, wherein the L-glutamine is ingested for a period of at least six months.

6. L-glutamine for use in a method according to claim 5, wherein the number of diverticula in the person with diverticulosis is reduced by at least fifty percent.

7. A composition comprising L-glutamine or an L-glutamine salt and a fiber supplement for use according to any one of claims 1-6.

8. The composition for use according to claim 7, wherein the composition comprises L-glutamine.

9. The composition for use according to claim 8, wherein the fiber is selected from a group of fibers consisting of methylcellulose, wheat dextrin and psyllium.

10. The composition for use according to claim 9, wherein the composition further comprises a flavorant.

11. The composition for use according to claim 10, wherein it is in the form of a powder, a capsule, a caplet or a chewable tablet.

## Patentansprüche

1. L-Glutamin oder ein L-Glutaminsalz zur Verwendung in einem Verfahren zur Behandlung von Divertikulose, wobei das Verfahren die Einnahme von 0,05 g/kg Körpergewicht bis 10,0 g/kg Körpergewicht L-Glutamin oder L-Glutaminsalz pro Tag durch eine Person, die Divertikulose hat, umfasst, wobei das L-Glutamin oder L-Glutaminsalz einmal pro Tag verabreicht wird.

2. L-Glutamin oder ein L-Glutaminsalz zur Verwendung in einem Verfahren zur Behandlung von Divertikulose, wobei das Verfahren die Einnahme von 0,05 g/kg Körpergewicht bis 10,0 g/kg Körpergewicht L-Glutamin oder L-Glutaminsalz pro Tag durch eine Person, die Divertikulose hat, umfasst, wobei das L-Glutamin oder L-Glutaminsalz mehr als einmal pro Tag verabreicht wird.

3. L-Glutamin zur Verwendung in einem Verfahren nach Anspruch 1 oder Anspruch 2.

4. L-Glutamin zur Verwendung in einem Verfahren nach Anspruch 3, wobei das L-Glutamin als Teil einer wässrigen Lösung oder Suspension eingenommen wird.

5. L-Glutamin zur Verwendung in einem Verfahren nach Anspruch 4, wobei das L-Glutamin über einen Zeitraum von mindestens sechs Monaten eingenommen wird.

6. L-Glutamin zur Verwendung in einem Verfahren nach Anspruch 5, wobei die Anzahl der Divertikel bei der Person mit Divertikulose um mindestens fünfzig Prozent reduziert wird.

7. Zusammensetzung, umfassend L-Glutamin oder ein L-Glutaminsalz und einen Faserzusatz zur Verwendung nach einem der Ansprüche 1-6.

8. Zusammensetzung zur Verwendung nach Anspruch 7, wobei die Zusammensetzung L-Glutamin umfasst.

9. Zusammensetzung zur Verwendung nach Anspruch 8, wobei die Faser aus einer Gruppe von Fasern ausgewählt ist, die aus Methylcellulose, Weizendextrin und Psyllium besteht.

10. Zusammensetzung zur Verwendung nach Anspruch 9, wobei die Zusammensetzung ferner einen Geschmacksstoff umfasst.

11. Zusammensetzung zur Verwendung nach Anspruch 10, wobei sie in Form eines Pulvers, einer Kapsel, eines Caplets oder einer Kautablette vorliegt.

## Revendications

1. L-glutamine ou sel de L-glutamine destiné(e) à être utilisé(e) dans un procédé de traitement de la diverticulose, où le procédé comprend l'étape consistant à ingérer 0,05 g/kg de poids corporel à 10,0 g/kg de poids corporel de la L-glutamine ou du sel de L-glutamine par jour par une personne atteinte de diverticulose, où la L-glutamine ou le sel de L-glutamine est administré(e) une fois par jour.

2. L-glutamine ou sel de L-glutamine destiné(e) à être utilisé(e) dans un procédé de traitement de la diverticulose, où le procédé comprend l'étape consistant à ingérer 0,05 g/kg de poids corporel à 10,0 g/kg de poids corporel de la L-glutamine ou du sel de L-glutamine par jour par une personne atteinte de diverticulose, où la L-glutamine ou le sel de L-glutamine est administré(e) plus d'une fois par jour.

3. L-glutamine destinée à être utilisée dans un procédé selon la revendication 1 ou 2.

4. L-glutamine destinée à être utilisée dans un procédé selon la revendication 3, dans laquelle la L-glutamine est ingérée en tant que partie d'une solution ou suspension aqueuse.

5. L-glutamine destinée à être utilisée dans un procédé selon la revendication 4, dans laquelle la L-glutamine est ingérée pendant une période d'au moins six mois.

6. L-glutamine destinée à être utilisée dans un procédé selon la revendication 5, dans laquelle le nombre de diverticules chez la personne atteinte de diverticulose est réduit d'au moins cinquante pour cent.

7. Composition comprenant de la L-glutamine ou un sel de L-glutamine et un supplément de fibres destinée à être utilisée selon l'une quelconque des revendications 1 à 6.

8. Composition destinée à être utilisée selon la revendication 7, dans laquelle la composition comprend de la L-glutamine.

9. Composition destinée à être utilisée selon la revendication 8, dans laquelle la fibre est choisie dans un groupe de fibres consistant en la méthylcellulose, la dextrine de blé et le psyllium.

10. Composition destinée à être utilisée selon la revendication 9, dans laquelle la composition comprend en outre un aromatisant.

11. Composition destinée à être utilisée selon la revendication 10, dans laquelle elle se présente sous la forme d'une poudre, d'une gélule, d'un comprimé-capsule ou d'un comprimé à croquer.
